# EUROPEAN PATENT SPECIFICATION

(11) **EP 1 638 524 B1**
(45) Date of publication and mention of the grant of the patent: **08.09.2010**
(21) Application number: 04757447.0
(22) Date of filing: 02.07.2004
(51) Int. Cl.: A61K 9/00, A61K 9/70, A61K 36/61, A61P 27/16

(54) **COMPOSITION AND METHOD FOR THE TREATMENT OF WATER RELATED EAR DISORDERS**
ZUSAMMENSETZUNG UND VERFAHREN ZUR BEHANDLUNG VON WASSERBEDINGTEN OHRERKRANKUNGEN
COMPOSITION ET PROCEDE POUR TRAITER DES TROUBLES AUDITIFS PROVOQUES PAR L'EAU

(30) Priority: 02.07.2003 ZA 200305160
(43) Date of publication of application: 29.03.2006
(73) Proprietor: Swimseal International (Pty) Ltd., Lynnwood Pretoria, Gauteng (ZA)
(72) Inventor: Tunguy-Desmarais, Peter, Durban North 4051, KwaZulu-Natal (ZA)
(74) Representative: Marshall, John Grahame
(86) International application number: PCT/ZA2004/000074
(87) International publication number: WO 2005/002543

(56) References cited:
- EP-A- 0 576 748
- WO-A-98/29085
- GB-A- 965 236
- US-B1- 6 521 213
- ALTMAN P M: "AUSTRALIAN TEA TREE OIL-A NATURAL ANTISEPTIC" AUSTRALIAN JOURNAL OF BIOTECHNOLOGY, vol. 3, no. 4, October 1989 (1989-10), pages 247-248, XP000783533 ISSN: 0819-3355

## Description

### Background to the invention

This invention relates to ear drops for use in the prevention or treatment of water related ear canal disorders.

### Summary of the invention

According to this invention ear drops for the prevention or treatment of water related outer ear canal are presented in a form suitable for applying topically to the external ear canal.

The invention provides ear drops for use in the treatment of water related outer ear canal disorders as defined in claim 1 herein. The ear drops are capable of being instilled in the outer ear canal in droplet form.

The polysiloxane is present as a water repellent agent in the ear drops according to the invention. The polysiloxane may be a water repellent silicone polymer adapted, in combination with the Tea Tree oil or on its own, to constitute the film forming liquid. Polysiloxanes are a group of silicone oils which are biologically inert, water repellent, film forming liquids.

The Tea Tree oil is an antimicrobial agent which is a natural product capable of acting against a broad range of bacterial and fungal infections. It is a natural antiseptic.

The two oils combine to produce a film forming liquid that is capable of being applied topically to the external ear canal by instillation in droplet form.

### Description of embodiments of the invention

The physiology of the skin lining the external ear canal is well known to otological clinicians, but for some reason, the application of a physiological basis to the pharmacological treatment of water related outer ear canal disorders seems to have been neglected.

A normal, healthy human outer ear canal is lined with cerumen glands that secrete a waxy exudate known as cerumen which aids in trapping airborne debris, repelling water and acidifying the epithelial surface of the canal. This assists in minimising bacterial and fungal infections within the canal. Fine hairs within the outer ear canal impel the ear wax gradually outwardly, carrying any dirt it has trapped. Eventually, the ear wax migrates out of the outer ear canal, dries and falls out of the ear in small, unnoticeable flakes.

Inflammation of the outer ear canal often arises as a result of improper ear care or when too much water gets in the ear. The warm, humid environment of the outer ear canal and the removal of protective ear wax during swimming, showering or over-enthusiastic cleaning combine to provide ideal conditions for the proliferation of harmful bacteria and fungi. In addition, exposure to large amounts of water such as, for instance, during swimming, results in the epithelial lining of the outer ear canal becoming more alkaline, leading to an increased growth in bacteria, fungal and viral organisms which capitalise on the more alkaline conditions.

The remedies conventionally prescribed for avoiding the ingress of water into the outer ear canal include the use of ear plugs of various types to prevent the ingress of water or the avoidance of water altogether.

An alternative to ear plugs is to apply petroleum jelly to the ear canal as a means of plugging the canal: This often results in the formation of a matrix with desquamating skin within the canal, further encouraging water retention in the ear canal.

The remedies conventionally prescribed for removing excess water from the outer ear canal include the use of hairdryers to dry the ear after the ingress of water or instiling an alcohol containing drop to disperse water. This is the most favoured approach.

Both these remedies have their place in the treatment of water related outer ear canal disorders, but it is generally accepted that the use of alcohol drops in particular has a serious shortcoming. Whilst assisting in water dispersion and drying of the skin of the outer ear canal, alcohol tends to cause excessive stripping of cerumen which leaves the skin excessively dry and subject to the effects of humidity and water. Upon further exposure to moisture, the skin becomes macerated and soggy, and encourages the growth of bacteria and fungi.

In contrast, the ear drops of this invention consist of a film forming liquid comprising a water repellent agent (the polysiloxane) and an antimicrobial agent (the Tea Tree oil). The film forming liquid is capable of being instilled in the outer ear canal in droplet form.

The ear drops may conveniently comprise between 75% and 98% by volume of a polysiloxane and between 2% and 25% by volume of Tea Tree oil.

The ear drops of this invention are adapted to seal the skin of the outer ear canal with a water repelling layer of the polysiloxane whilst bringing to bear the natural antimicrobial action of Tea Tree oil.

The polysiloxane is inert and exerts its action purely because of its physical properties. It is preferably used in the composition as a relatively thin, non-viscous oil. In this form, it has good film-forming properties on human skin. Instead of the physically invasive use of ear plugs or the equally ineffectual use of petroleum jelly, the polysiloxane forms a thin, chemically inert, water repellent film on the skin of the outer ear canal.

Tea Tree (*Melaleuca alternifolia*) and particularly its essential oil are widely regarded as some of the most important natural antibacterial and antiseptic treatments. Tea Tree oil is used for treating stings, burns, wounds and skin infections of all kinds. The essential oil, which is obtained from the leaves and twigs of the plant, is strongly antiseptic, diaphoretic and expectorant. It stimulates the immune system and is effective against a broad range of bacterial and fungal infections. The essential oil of *Melaleuca alternifolia* is non-irritant and it is used externally in the treatment of a number of skin disorders, for which uses it is normally diluted with a carrier oil such as almond oil.

In the ear drops of the invention, the Tea Tree oil is used as the essential oil of *Melaleuca alternifolia*, diluted with polysiloxane.

In use, a person hoping to prevent water related ear problems will instill a few drops of the composition in both ears a short while before entering water. A person who regularly experiences such ear problems could also use the ear drops more regularly, between two and four times a week, for instance, as a preventative measure. Being largely natural and devoid of any irritants, preservatives, alcohol or other pharmacologically active substances, the ear drops will be well tolerated in such a preventative treatment regime.

## Claims

1. Ear drops for use in the prevention or treatment of water related outer ear canal disorders, consisting of a film forming liquid comprising a polysiloxane and Tea Tree oil (the essential oil of the Tea Tree (*Melaleuca alternifolia*)).

2. Ear drops according to claim 1, comprising from 75% to 98% by volume of a polysiloxane and from 2% to 25% by volume of Tea Tree oil.

3. Ear drops for use in the prevention or treatment of water related outer ear canal disorders, consisting of a water repellent agent in the form of a polysiloxane and an antimicrobial agent in the form of the essential oil of *Melaleuca alternifolia* or Tea Tree oil in which the two oils are combined to produce a film forming liquid that is capable of being applied topically to the external ear canal by instillation in droplet form.

4. The use of a film forming liquid comprising a combination of a polysiloxane and Tea Tree oil (the essential oil of the Tea Tree (*Melaleuca alternifolia*)) in the manufacture of ear droplets for use in a method for the prevention or treatment of water related outer ear canal disorders.

## Patentansprüche

1. Ohrentropfen zur Verwendung bei der Vorbeugung oder Behandlung von wasserbedingten äußeren Gehörkanalstörungen, bestehend aus einer Film bildenden Flüssigkeit, mit einem Polysiloxan und Teebaumöl (dem ätherischen Öl des Teebaums (*melaleuca alfernifolia*)).

2. Ohrentropfen nach Anspruch 1, mit einer Volumenverteilung von 75 % - 98 % eines Polysiloxans und von 2% bis 25 % Teebaumöl.

3. Ohrentropfen zur Verwendung in der Vorbeugung oder Behandlung von wasserbedingten äußeren Gehörkanalstörungen, bestehend aus einem wasserabstoßendem Wirkstoff in der Form eines Polysiloxans und eines antimikrobiellen Wirkstoffs in Form des ätherischen Öls von *melaleuca alternifolia* oder Teebaumöl, bei dem die beiden Öle kombiniert sind, um eine Film bildende Flüssigkeit zu bilden, welche zum örtlichen Auftragen auf den äußeren Ohrkanal über Einträufelung in Tröpfchenform geeignet ist.

4. Verwendung einer Film bildenden Flüssigkeit, mit einer Kombination eines Polysiloxans und Teebaumöl (dem ätherischen Öl des Teebaums (*melaleuca alternifolia*)), bei der Herstellung von Ohrentropfen zur Verwendung bei einem Verfahren für die Vorbeugung oder Behandlung von wasserbedingten äußeren Gehörkanalstörungen.

## Revendications

1. Gouttes auriculaires utilisables pour la prévention ou le traitement de troubles du canal auditif externe liés à l'eau, consistant en un liquide formant un film comprenant un polysiloxane et de l'huile de l'arbre à thé (l'huile essentielle de l'arbre à thé (*Melaleuca alternifolia*)).

2. Gouttes auriculaires selon la revendication 1, comprenant de 75 % à 98 % en volume d'un polysiloxane et de 2 % à 25 % en volume d'huile de l'arbre à thé.

3. Gouttes auriculaires utilisables pour la prévention ou le traitement de troubles du canal auditif externe liés à l'eau, consistant en un agent hydrorépulsif sous la forme d'un polysiloxane et d'un agent antimicrobien sous la forme de l'huile essentielle de *Melaleuca alternifolia* ou huile de l'arbre à thé dans lequel les deux huiles sont combinées pour produire un liquide formant un film convenant pour une application topique dans le canal auditif externe par instillation sous forme de gouttelettes.

4. Utilisation d'un liquide formant un film comprenant une combinaison d'un polysiloxane et d'huile de l'arbre à thé (l'huile essentielle de l'arbre à thé (*Melaleuca alternifolia*)) dans la préparation de gouttelettes auriculaires utilisables pour la prévention ou le traitement de troubles du canal auditif externe liés à l'eau.
